# EUROPÄISCHE PATENTSCHRIFT

(11) **EP 0 366 092 B1**
(45) Veröffentlichungstag und Bekanntmachung des Hinweises auf die Patenterteilung: **02.03.1994**
(21) Anmeldenummer: 89119758.4
(22) Anmeldetag: 24.10.1989
(51) Int. Cl.: G01N 33/68, G01N 33/543, G01N 33/538, G01N 33/576

(54) **Verfahren zur Bestimmung von für ein Antigen klassenspezifischen Antikörpern und dazu geeignetes Reagenz**
Method for the detection of antigen and class specific antibodies and reagent therefor
Procédé pour déterminer des anticorps antigène- et classes-spécifiques et réactif pour ce procédé

(30) Priorität: 25.10.1988 DE 3836348
(43) Veröffentlichungstag der Anmeldung: 02.05.1990
(73) Patentinhaber: BOEHRINGER MANNHEIM GMBH, 68298 Mannheim (DE)
(72) Erfinder: Schmitt, Urban, D-8125 Oberhausen (DE)
(74) Vertreter: Huber, Bernhard, Dipl.-Chem.

(56) Entgegenhaltungen:
- EP-A- 0 005 271
- GB-A- 2 026 691
- GB-A- 2 203 836
- US-A- 4 020 151
- JOURNAL OF CLINICAL MICROBIOLOGY, vol. 24, no. 2, August 1986, Washington, DC (US); W.F. GERLICH et al., Seiten 288-293#
- CLINICAL CHEMISTRY, vol. 30, no. 7, Juli 1984, Winston (US); J.A. HINDS et al., Seiten 1174-1178#

## Beschreibung

Die Erfindung betrifft ein Verfahren zur Bestimmung von für ein Antigen klassenspezifischen Antikörpern in Körperflüssigkeiten nach dem Prinzip des Immunoassay durch Inkubation der Probelösung mit mindestens zwei Rezeptoren R¹ und R² von denen R¹ immobilisierbar und mit dem zu bestimmenden Antikörper bindefähig ist und R² markiert und entweder mit dem zu bestimmenden Antikörper oder mit dem für den zu bestimmenden Antikörper spezifischen Antigen bindefähig ist, Trennung der festen von der flüssigen Phase und Messung der Markierung in einer der beiden Phasen sowie ein dazu geeignetes Reagenz.

Der Körper produziert als Antwort auf das Einbringen fremder Substanzen Antikörper, die auch als Immunglobuline bezeichnet werden. Die Immunglobuline können in 5 Klassen aufgeteilt werden, die sich durch ihren Aufbau unterscheiden. Die verschiedenen Immunglobulin-Klassen haben im Organismus verschiedene Aufgaben. So werden bei einer Erstimmunisierung zuerst Immunglobuline der Klasse M gebildet, deren Konzentration jedoch bei fortschreitender Infektion schnell wieder abnimmt. Die Immunglobuline der Klasse G werden bei einer ersten Immunisierung erst langsam gebildet und treten bei einer zweiten Reaktion mit demselben Antigen in großen Mengen auf. Die Immunglobuline der Klasse A sind für Abwehrvorgänge an den Schleimhautoberflächen des Organismus zuständig. Die Immunglobuline der Klasse E sind hauptsächlich für allergische Reaktionen verantwortlich. Über die Immunglobuline der Klasse D ist bisher relativ wenig bekannt. Die einzelnen Immunoglobulinklassen kommen im Blut in sehr unterschiedlichen Konzentrationen vor. So liegt der IgG-Gehalt im Serum normaler Personen im Bereich von 8 bis 16 mg/l. Antikörper der IgM-Klasse sind im Serum in einer Menge von 0,5 bis 2 mg/ml enthalten. Das am zweithäufigsten vorkommende Immunglobulin ist das IgA, das im Serum normaler Personen im Bereich von 1,4 bis 4 mg/ml enthalten ist. Am geringsten ist die Konzentration von IgE im Serum, die im Bereich von 0,000017 bis 0,00045 mg/ml liegt. Der Gehalt an IgD im Serum liegt im Bereich von 0 bis 0,4 mg/ml.

Für die Diagnostik ist es nun interessant, Antikörper einer bestimmten Immunglobulinklasse, die für ein bestimmtes Antigen spezifisch sind, nachzuweisen, da auf diese Weise eine Differentialdiagnose bei viralen, bakteriellen und parasitären Infektionen gestellt werden kann. So eignet sich insbesondere die IgG- und IgM-Bestimmung zur Unterscheidung frischer und weit zurückliegender Infektionen sowie zur Kontrolle des Verlaufs einer Infektion. Insbesondere werden klassenspezifische Antikörper nachgewiesen bei Hepatitis B-, Toxoplasmose-, Rubella- und Chlamydia-Infektionen.

Verfahren zur Bestimmung von klassenspezifischen Antikörpern sind bekannt. So wird beispielsweise in US-PS 4,020,151 ein Verfahren zur Bestimmung der IgG-, IgA- und IgM-Konzentrationen im Serum beschrieben, bei dem zunächst ein fester Träger mit der Probelösung umgesetzt wird, wobei IgG, IgA oder IgM an der Festphase adsorbiert werden und anschließend wird markierter Antikörper gegen die jeweilige Ig-Klasse zugegeben und die gebundene Markierung ausgewertet. Ein anderes Nachweissystem ist bei W. Duermeyer et al., The Lancet II, 684-685 beschrieben, wobei ein Antikörper für eine spezifische Immunglobulinklasse an einen festen Träger gebunden wird. Nach Zugabe der Probe werden die klassenspezifischen Immunglobuline an die Wand gebunden und in einem zweiten Schritt wird das für den zu bestimmenden Antikörper spezifische Antigen zugegeben, das dann an die Antikörper bindet. In einem dritten Schritt wird ein gegen dieses Antigen gerichteter markierter Antikörper zugegeben und wiederum der Anteil an gebundener Markierung bestimmt.

Diese bisher bekannten Verfahren zur Bestimmung klassenspezifischer Immunglobuline sind jedoch noch nicht befriedigend. Wegen der begrenzten Bindekapazität der Festphase und der hohen Analytkonzentration in der Probe kann nur ein kleiner Anteil der im Serum enthaltenen Immunglobulinklassen gebunden werden. Es kommt daher zu starken Schwankungen bei der quantitativen Bestimmung der antigenspezifischen Immunglobuline, da der Gesamtgehalt dieser Immunglobuline im Serum starken Schwankungen unterliegt. Es kommt daher mit den bekannten Verfahren bei gleichem antigenspezifischem Immunglobulingehalt einer Immunglobulinklasse bei verschiedenen Proben zu unterschiedlichen Meßergebnissen, so daß im Extremfall eine tatsächlich positive Probe als negativ nachgewiesen wird.

Um diese Störung zu verringern wird beispielsweise in Journal of Clinical Microbiology, August 1986, Seiten 288 bis 293 vorgeschlagen, die Probelösung zu verdünnen, anschließend ein Serum, das Antikörper der zu bestimmenden Immunglobulinklasse enthält, zuzugeben und dann weiter zu verdünnen. Bei diesem Verfahren sind Verdünnungen von 1:2000 bis 1:20000 notwendig. Einerseits wird durch diese starke Verdünnung die Reproduzierbarkeit der erhaltenen Ergebnisse in Frage gestellt und andererseits ist dieses Verfahren nicht auf Analyseautomaten übertragbar.

Aufgabe der vorliegenden Erfindung war es daher, ein Verfahren zur Verfügung zu stellen, bei dem für ein Antigen klassenspezifische Antikörper in Körperflüssigkeiten quantitativ bestimmt werden können, ohne daß eine umständliche Probenvorbereitung oder hohe Verdünnung der Probe notwendig ist und das mit wenigen Stufen durchführbar ist.

Diese Aufgabe wird gelöst durch ein Verfahren zur Bestimmung von Antikörpern einer bestimmten Immunglobulinklasse, die gegen ein Antigen A gerichtet sind, in Körperflüssigkeiten nach dem Prinzip des Immunoassay durch Inkubation der Probelösung mit mindestens zwei Rezeptoren R¹ und R², von denen R¹ immobilisierbar ist und mit dem zu bestimmenden Antikörper bindefähig ist und R² markiert ist und entweder mit dem zu bestimmenden Antikörper oder dem Antigen A bindefähig ist, Trennung der festen von der flüssigen Phase und Messung der Markierung in einer der beiden Phasen, das dadurch gekennzeichnet ist, daß man die immunologische Reaktion in homogener Phase durchführt und die Probelösung gleichzeitig mit Rezeptor R¹ und einem Überschuß an humanen Antikörpern oder deren Fragmenten, die der gleichen Immunglobulinklasse angehören, wie die zu bestimmenden Antikörper und nicht mit dem Antigen A bindefähig sind, inkubiert und anschließend Rezeptor R² und gegebenenfalls weitere Rezeptoren zugibt.

Überraschenderweise gelingt es mit dem erfindungsgemäßen Verfahren, klassenspezifische Antikörper reproduzierbar nachzuweisen, ohne daß eine Vorbehandlung der Probe erfolgen muß. Das erfindungsgemäße Verfahren ist einfach und schnell durchzuführen.

Erfindungsgemäß werden die für ein Antigen A klassenspezifischen Antikörper in einem homogenen Immunoassay bestimmt, d.h., daß die immunologische Reaktion in homogener Phase abläuft. Dazu wird als Rezeptor R¹, der mit dem zu bestimmenden Antikörper bindefähig ist, ein immobilisierbarer Rezeptor eingesetzt, der während der immunologischen Reaktion in homogener Phase vorliegt und anschließend an einer Festphase immobilisiert werden kann. Rezeptor R¹ enthält daher einen Anteil, der die Bindung an die feste Phase ermöglicht und andererseits einen Anteil, der die Bindung mit dem klassenspezifischen Antikörper ermöglicht. Zur Bindung mit dem klassenspezifischen Antikörper wird bevorzugt ein gegen den konstanten Teil des Antikörpers gerichteter Antikörper oder dessen Fragment oder Derivat verwendet. Sollen also Immunglobuline der Klasse G bestimmt werden, so enthält der Rezeptor R¹ Anti-IgG- Antikörper oder ein Fab-Fragment davon. Zur Immobilisierung kann entweder ein mit dem konstanten Teil von Rezeptor R¹ bindefähiger Rezeptor an der Festphase immobilisiert sein. Bevorzugt wird jedoch als Rezeptor R¹ ein Konjugat aus mit dem klassenspezifischen Antikörper bindefähigen Anteil und einem Partner eines spezifisch bindefähigen Paares, besonders bevorzugt Biotin, verwendet. Als Festphase kann dann eine Matrix verwendet werden, an der der andere Partner des spezifisch bindenden Paares, besonders bevorzugt Streptavidin, gebunden ist. Die Immobilisierung erfolgt dann durch Bindung der beiden Partner miteinander. Da diese Bindung heterogen erfolgt und damit sehr viel langsamer ist als die immunologische Reaktion, beeinträchtigt dies die Durchführung der immunologischen Reaktion in homogener Phase nicht. In einer anderen Variante kann an die Festphase ebenfalls derselbe Partner des spezifisch bindenden Paares gebunden sein, bevorzugt Biotin, wobei dann zur Immobilisierung der andere Partner des spezifisch bindenden Paares zugegeben wird, der dann die Bindung zwischen Festphase und Rezeptor R¹ vermittelt.

Zur Durchführung des Verfahrens wird zunächst zu Rezeptor R¹ und der Probelösung ein Überschuß an humanem klassenspezifischem Antikörper der zu bestimmenden Immunglobulinklasse, der nicht mit dem Antigen A bindefähig ist oder dessen Fragment zugesetzt. Als Fragmente können dabei die Fc-Fragmente der zu bestimmenden Immunglobulin-Klasse oder aber die H-Ketten Antigendeterminante verwendet werden. So kann zum Nachweis von IgM die µ-Kette, zum Nachweis von IgG die γ-Kette usw. eingesetzt werden.

Bevorzugt wird der humane klassenspezifische Antikörper in einem Überschuß von 2 bis 100:1, bezogen auf die in der Probelösung zu erwartende Menge an Immunglobulin eingesetzt.

Bevorzugt wird zuerst eine Lösung aus Rezeptor R¹ und dem Überschuß des klassenspezifischen Antikörpers gebildet. Dabei reagiert der mit dem zu bestimmenden Antikörper bindefähige Anteil des Rezeptors R1 mit den zugegebenen klassenspezifischen Antikörpern. Anschließend wird die Probelösung zugegeben. Die nun im Unterschuß in der Probelösung vorliegenden klassenspezifischen Antikörper verdrängen zum Teil die im Überschuß vorliegenden unspezifischen Antikörper aus dem Komplex. Dabei läuft diese Verdrängungsreaktion umso rascher ab, je geringer die Aktivität des klassenspezifischen Antikörpers zu R¹ ist. Vorzugsweise werden deshalb klassenspezifische Antikörper mit einer Aktivität zu R¹ von <5·10⁻¹¹ l/mol eingesetzt. Nach anschließender Zugabe von Rezeptor R² bindet dieser an den Komplex aus zu bestimmendem Antikörper und Rezeptor R¹. Nach Immobilisierung kann dann der Anteil an gebundener Markierung in an sich bekannter Weise bestimmt werden und ist ein direktes Maß für die Menge an klassenspezifischem Antikörper in der Probelösung.

Die Menge an zugesetztem klassenspezifischem Antikörper ist nicht kritisch. Sie sollte mindestens doppelt so groß sein, wie der obere Grenzwert, der normalerweise für Immunglobuline dieser Klasse im Serum erwartet werden kann. Der Überschuß beträgt daher bevorzugt 2:1 bis 100:1, bezogen auf die zu erwartende Immunglobulinmenge. Bei einem Überschuß von mehr als dem 100fachen ergibt sich keine weitere Verbesserung mehr, so daß ein noch höherer Überschuß unwirtschaftlich ist.

Als zweiter Rezeptor wird ein markierter Rezeptor eingesetzt. Die Markierung erfolgt in an sich bekannter Weise und kann ein radioaktives Isotop, eine chemilumineszente oder fluoreszierende Substanz, eine farbstoffbildende Substanz oder ein Enzym sein. Ebenso sind Verfahren zum Nachweis dieser Markierungen bekannt und brauchen nicht näher erläutert zu werden. Rezeptor R² ist entweder mit dem zu bestimmenden Antikörper oder mit dem für diesen Antikörper spezifischen Antigen bindefähig. In der ersten Variante wird bevorzugt als Rezeptor R² das für den zu bestimmenden Antikörper spezifische Antigen verwendet, das dann in an sich bekannter Weise markiert ist. In der anderen Variante wird als Rezeptor R² bevorzugt ein gegen das für den zu bestimmenden Antikörper spezifische Antigen gerichteter Antikörper oder dessen Fragment verwendet. Der Antikörper kann dabei monoklonal oder polyklonal sein. In dieser zweiten Ausführungsform ist dann für die Bestimmung noch ein weiterer Rezeptor R³ erforderlich, der eine mit dem zu bestimmenden Antikörper und mit R² spezifisch bindefähige Substanz ist. Besonders bevorzugt enthält R³ das für den zu bestimmenden Antikörper spezifische Antigen. Das Antigen kann unmittelbar als R³ eingesetzt werden, wenn es Bindungsstellen (Epitope) für den zu bestimmenden Antikörper und Rezeptor R² trägt.

Zur Durchführung des erfindungsgemäßen Verfahrens wird die Probelösung zuerst mit Rezeptor R¹ und dem Überschuß an klassenspezifischem Antikörper inkubiert und anschließend Rezeptor R² zugesetzt. Bei der Verfahrensvariante, bei der Rezeptor R³ verwendet wird, wird dieser vor der Zugabe von Rezeptor R² der Probelösung zugegeben.

Besonders bevorzugt ist die Durchführungsform des erfindungsgemäßen Verfahrens, bei der nur zwei Rezeptoren erforderlich sind, so daß die Reaktion in zwei Schritten durchgeführt werden kann und damit einer Übertragung auf einen Analyseautomaten zugänglich ist.

Die immunologische Reaktion erfolgt in homogener Phase, was sehr vorteilhaft ist, da die homogene Reaktion schneller und reproduzierbarer verläuft.

Gegenstand der Erfindung ist auch ein Reagenz zur Bestimmung von für ein Antigen klassenspezifischen Antikörpern in Körperflüssigkeiten, das Rezeptor R¹, der immobilisierbar ist und mit dem zu bestimmenden Antikörper bindefähig ist und einem Überschuß an humanen klassenspezifischen Antikörpern, die nicht mit dem Antigen A binden können, und als Lösung 2 Rezeptor R², der markiert ist und das Antigen A aufweist, enthält.

In einer weiteren Variante enthält das erfindungsgemäße Reagenz als Lösung 1 eine Mischung von Rezeptor R¹ und einem Überschuß an humanen klassenspezifischen Antikörpern, als Lösung 2 Rezeptor R², der markiert ist, und einen gegen Antigen A gerichteten Antikörper enthält und als Lösung 3 Rezeptor R³, der das Antigen A aufweist.

In dieser Variante muß Rezeptor R³ mindestens je eine Bindestellen für den spezifisch bindefähigen Antikörper und für Rezeptor R² aufweisen. Es werden deshalb entweder für Rezeptor R³ Antigene verwendet, die an sich polyvalent sind oder aber, falls ein Antikörper nachgewiesen werden soll gegen ein Antigen, das monovalent ist, wird für Rezeptor R³ das Antigen in polymerer Form verwendet. Hierzu kann das Antigen an Träger gebunden werden wie z.B. RSA, Latex oder Mikrocarrier oder quervernetzt werden. Verfahren hierzu sind dem Fachmann bekannt.

In einer bevorzugten Ausführungsform können Lösung 2 und Lösung 3 des Reagenzes vorgemischt werden. Das Reagenz enthält dann als Lösung 1 eine Mischung von Rezeptor R¹ und einem Überschuß an humanen klassenspezifischen Antikörpern und als Lösung 2 Rezeptor R², der markiert ist und einen gegen Antigen A gerichteten Antikörper enthält und Rezeptor R³, der das Antigen A aufweist, enthält.

Das Reagenz enthält den humanen klassenspezifischen Antikörper in einem solchen Überschuß, daß bezogen auf die in der Probelösung zu erwartende Menge an Immunglobulin die 2- bis 100-fache Menge an humanem klassenspezifischen Antikörper in der Reaktionslösung vorhanden ist. Dazu enthält das Reagenz für den Fall, daß 10 µl Probe zu 1 ml Reagenz zugegeben werden, bevorzugt ca. 20 bis 2000 mg Antikörper pro Liter Reagenz.

Das erfindungsgemäße Reagenz enthält in zwei bzw. drei getrennten Lösungen die zur Bestimmung von klassenspezifischen Antikörpern notwendigen Komponenten. Die Lösungen werden physikalisch getrennt voneinander aufbewahrt und zur Bestimmung nacheinander mit der Probelösung inkubiert. Die Lösungen sind lagerstabil.

Die Erfindung wird durch die folgenden Beispiele erläutert.

### Beispiel 1

Es wurden Anti-HBcAg-Antikörper der Immunglobulinklasse M in Serum bestimmt. Dazu wurde als Festphase ein mit Streptavidin beschichtetes Röhrchen verwendet. In dieses Röhrchen wurden 10 µl Probe zusammen mit 1000 µl eines Reagenzes der folgenden Zusammensetzung gegeben:
- Reagenz 1: 40 mmol/l Phosphatpuffer, pH 7,0, 0,5% Pluronic F68, 0,2 % Rinderserumalbumin, 0,01 % Phenol, 40 mg/l humanes IgM, das nicht mit HBcAg bindefähig ist, 2 mg/l eines monoklonalen Antikörpers gegen IgM, der mit Biotin gekoppelt wurde.

Die Inkubation erfolgte bei Raumtemperatur 30 Minuten. Nach dreimaligem Waschen mit Wasser wurde 1 ml eines Reagenzes der folgenden Zusammensetzung zugegeben:
- Reagenz 2: 40 mmol/l Phosphatpuffer, pH 7,0, 0,2 % Rinderserumalbumin, 0,01 % Phenol, 75 µg/l Hepatitis Core-Antigen, 25 U/l eines Konjugats aus einem monoklonalen Anti-HBCAg-Antikörper und Peroxidase.

Bei einer Inkubationsdauer von 60 Minuten bei 20 bis 22°C und dreimaligem Waschen mit Wasser wurde 1 ml ABTS-Lösung als Substrat zugegeben. Die Extinktion wurde nach einer Stunde bei 405 nm bestimmt und anhand einer Eichkurve ausgewertet. Die Ergebnisse sind der folgenden Tabelle zu entnehmen.

**Tabelle**

| Serum | ohne Human IgM im Reagenz | | mit Human IgM im Reagenz | |
|---|---|---|---|---|
| | Extinktion (mE) | % Abweichung vom Mittelwert¹⁾ | Extinktion (mE) | % Abweichung vom Mittelwert ²⁾ |
| 1 | 235 | 132 | 465 | 103 |
| 2 | 119 | 67 | 397 | 88 |
| 3 | 216 | 121 | 471 | 104 |
| 4 | 120 | 67,4 | 437 | 97 |
| 5 | 211 | 119 | 493 | 109 |
| 6 | 92 | 52 | 385 | 85 |
| 7 | 161 | 90 | 460 | 102 |
| 8 | 276 | 155 | 496 | 110 |
| 9 | 194 | 109 | 461 | 102 |
| 10 | 159 | 89 | 444 | 98 |

| | | | | |
|---|---|---|---|---|
| ¹⁾ Mittelwert: 179 | | | | |
| ²⁾ Mittelwert: 451 | | | | |

### Beispiel 2

Es wurde Anti-HBc-Antikörper der IgM-Klasse im Serum bestimmt analog Beispiel 1, wobei jedoch statt des biotinylierten monoklonalen Antikörpers gegen humanes IgM ein biotinylierter polyklonaler Anti-human-IgM-Antikörper in Reagenz 1 verwendet wurde. Die Auswertung erfolgte, wie im Beispiel 1 beschrieben.

### Beispiel 3

Es wurden Anti-HBcAg-Antikörper der Klasse IgM in Serum nachgewiesen, wie im Beispiel 1 beschrieben. Dabei wurde jedoch in Reagenz 2 statt des Antikörper POD-Konjugates und des Hepatitis Core-Antigen ein Konjugat aus HBcAg und Peroxidase verwendet. Die Auswertung erfolgte wie im Beispiel 1 beschrieben.

## Patentansprüche

1. Verfahren zur Bestimmung von Antikörpern einer bestimmten Immunglobulinklasse, die gegen ein Antigen A gerichtet sind, in Körperflüssigkeiten nach dem Prinzip des Immunoassay durch Inkubation der Probelösung mit mindestens zwei Rezeptoren R¹ und R², von denen R¹ immobilisierbar und mit dem zu bestimmenden Antikörper bindefähig ist und R² markiert ist und entweder mit dem zu bestimmenden Antikörper oder dem Antigen A bindefähig ist, Trennung der festen von der flüssigen Phase und Messung der Markierung in einer der beiden Phasen,
**dadurch gekennzeichnet,** daß man die immunologische Reaktion in homogener Phase durchführt und die Probelösung zuerst mit einer Reagenzlösung inkubiert, die gleichzeitig Rezeptor R¹ und einen Überschuß an humanen Antikörpern oder deren Fragmenten bezogen auf die in der Probenlösung zu erwortende Menge an Immunoglobulin, die der gleichen Immunglobulinklasse angehören wie die zu bestimmenden Antikörper und nicht mit dem Antigen A bindefähig sind, enthält und anschließend Rezeptor R² und gegebenenfalls weitere Rezeptoren zugibt, wobei wenn R² ein Antikörper ist, ein weiterer Rezeptor R³, der mindestens je eine Bindestelle für den zu bestimmenden Antikörper und den Rezeptor R² enthält, vor R² zu der Probelösung gegeben wird.

2. Verfahren nach Anspruch 1, **dadurch gekennzeichnet,** daß das Verfahren zur Bestimmung von IgM-Antikörpern verwendet wird.

3. Verfahren nach Anspruch 1 oder 2, **dadurch gekennzeichnet,** daß als Rezeptor R¹ ein Konjugat aus Biotin und einem Anti-IgM-Antikörper oder dessen Derivat und als Festphase eine Matrix verwendet wird, an der eine Vielzahl von Streptavidin-Molekülen gebunden sind.

4. Verfahren nach einem der vorhergehenden Ansprüche, **dadurch gekennzeichnet,** daß als Rezeptor R² ein Konjugat aus einer Markierung und Antigen A verwendet wird.

5. Verfahren nach einem der Ansprüche 1 bis 3, **dadurch gekennzeichnet,** daß als Rezeptor R² ein Konjugat aus einem gegen Antigen A gerichteten Antikörper und einer Markierung und als Rezeptor R³ Antigen A verwendet wird.

6. Verfahren nach einem der vorhergehenden Ansprüche, **dadurch gekennzeichnet,** daß als humaner klassenspezifischer Antikörper, Fcγ-Fragmente oder die jeweiligen H-Ketten eingesetzt werden.

7. Verfahren nach einem der vorhergehenden Ansprüche, **dadurch gekennzeichnet,** daß der humane klassenspezifische Antikörper in einem Überschuß von 2:1 bis 100:1, bezogen auf die in der Probelösung zu erwartende Menge an Immunglobulin, verwendet wird.

8. Reagenz zur Bestimmung von Antikörpern einer bestimmten Immunglobulinklasse nach einem der Ansprüche 1 bis 7, **dadurch gekennzeichnet,** daß es als Lösung 1 eine Mischung von Rezeptor R¹, der immobilisierbar ist und mit dem zu bestimmenden Antikörper bindefähig ist und einem Überschuß an humanen klassenspezifischen Antikörpern, die nicht mit dem Antigen A binden können, und als Lösung 2 Rezeptor R², der markiert ist und das Antigen A aufweist, enthält.

9. Reagenz, **dadurch gekennzeichnet,** daß es als Lösung 1 eine Mischung von Rezeptor R¹ und einem Überschuß bezogen auf die in der Probenlösung zu erwortende Menge an Immunoglobulin an humanen klassenspezifischen Antikörpern, als Lösung 2 Rezeptor R², der markiert ist und einen gegen Antigen A gerichteten Antikörper enthält und als Lösung 3 Rezeptor R³, der das Antigen A mit mindestens je einer Bindestelle für den zu bestimmenden Antikörper und den Rezeptor R² aufweist, enthält.

## Claims

1. Process for the determination in body fluids of antibodies of a definite immunoglobulin class which are directed against an antigen A according to the immunoassay principle by incubation of the sample solution with at least two receptors R¹ and R², of which R¹ is immobilisable and is bindable with the antibody to be determined and R² is labelled and is bindable either with the antibody to be determined or the antigen A, separation of the solid from the liquid phase and measurement of the labelling in one of the two phases, characterised in that one carries out the immunological reaction in homogeneous phase and incubates the sample solution first with a reagent solution which simultaneously contains receptor R¹ and an excess of human antibodies or their fragments, referred to the amount of immunoglobulin to be expected in the sample solution, which belong to the same immunoglobulin class as the antibody to be determined and are not bindable with the antigen A and subsequently adds thereto receptor R² and possibly further receptors, whereby, when R² is an antibody, a further receptor R³, which contains, in each case, at least one binding position for the antibody to be determined and the receptor R²_{,} is added to the sample solution before R².

2. Process according to claim 1, characterised in that the process is used for the determination of IgM antibodies.

3. Process according to claim 1 or 2, characterised in that, as receptor R¹, there is used a conjugate of biotin and an anti-IgM antibody or its derivatives and, as solid phase, a matrix to which are bound a plurality of streptavidin molecules.

4. Process according to one of the preceding claims, characterised in that, as receptor R², a conjugate is used of a labelling and antigen A.

5. Process according to one of claims 1 to 3, characterised in that, as receptor R², a conjugate is used of an antibody directed against antigen A and a labelling and, as receptor R³, antigen A.

6. Process according to one of the preceding claims, characterised in that, as human class-specific antibodies, Fcγ fragments are used of the H chains in question.

7. Process according to one of the preceding claims, characterised in that the human class-specific antibody is used in an excess of 2:1 to 100:1, referred to the amount of immunoglobulin to be expected in the sample solution.

8. Reagent for the determination of antibodies of a definite immunoglobulin class according to one of claims 1 to 7, characterised in that it contains a mixture of receptor R¹ which is immobilisable and is bindable with the antibody to be determined and an excess of human class-specific antibodies which cannot bind with anti-gen A and, as solution 2, receptor R² which is labelled and has the antigen A.

9. Reagent, characterised in that, as solution 1, it contains a mixture of receptor R¹ and an excess, referred to the amount of immunoglobulin to be expected in the sample solution, of human class-specific antibodies, as solution 2, receptor R² which is labelled and contains an antibody directed against antigen A and, as solution 3, receptor R³ which contains the antigen A with, in each case, at least one binding position for the antibody to be determined and the receptor R².

## Revendications

1. Procédé de détermination d'anticorps d'une classe d'immunoglobulines déterminée, qui sont dirigés contre un antigène A, dans les fluides corporels selon le principe de l'immuno-analyse, par incubation de la solution échantillon avec au moins deux récepteurs R¹ et R², parmi lesquels R¹ est immobilisable et capable de se lier à l'anticorps à déterminer et R² est marqué et est capable de se lier à l'anticorps à déterminer ou à l'antigène A, séparation de la phase solide et de la phase liquide et mesure du marqueur dans l'une des deux phases, caractérisé en ce que l'on réalise la réaction immunologique en phase homogène et on incube la solution échantillon d'abord avec une solution réactive qui contient simultanément le récepteur R¹ et un excès d'anticorps humains ou de leurs fragments, par rapport à la quantité d'immunoglobuline à laquelle on doit s'attendre dans la solution échantillon, qui appartiennent à la même classe d'immunoglobulines que les anticorps à déterminer et qui ne sont pas capables de se lier à l'antigène A, puis l'on ajoute le récepteur R² et éventuellement d'autres récepteurs, dans lequel lorsque R² est un anticorps, on ajoute à la solution échantillon, avant R², un autre récepteur R³ qui contient au moins un site de liaison pour l'anticorps à déterminer et un site de liaison pour le récepteur R².

2. Procédé selon la revendication 1, caractérisé en ce que le procédé est utilisé pour la détermination d'anticorps IgM.

3. Procédé selon la revendication 1 ou 2, caractérisé en ce que l'on utilise comme récepteur R¹ un conjugué de biotine et d'un anticorps anti-IgM ou de son dérivé et comme phase solide une matrice à laquelle sont liées une multiplicité de molécules de streptavidine.

4. Procédé selon l'une des revendications précédentes, caractérisé en ce que l'on utilise comme récepteur R² un conjugué d'un marqueur et d'antigène A.

5. Procédé selon l'une des revendications 1 à 3, caractérisé en ce que l'on utilise comme récepteur R² un conjugué d'un anticorps dirigé contre l'antigène A et d'un marqueur et comme récepteur R³ l'antigène A.

6. Procédé selon l'une des revendications précédentes, caractérisé en ce que l'on utilise comme anticorps humains de classe spécifique des fragments Fcγ ou les chaînes H correspondantes.

7. Procédé selon l'une des revendications précédentes, caractérisé en ce que l'anticorps humain de classe spécifique est utilisé en un excès de 2:1 à 100:1 par rapport à la quantité d'immunoglobuline à laquelle on doit s'attendre dans la solution échantillon.

8. Réactif de détermination d'anticorps d'une classe d'immunoglobulines déterminée selon l'une des revendications 1 à 7, caractérisé en ce qu'il contient comme solution 1 un mélange d'un récepteur R¹, qui est immobilisable et qui est capable de se lier à l'anticorps à déterminer et d'un excès d'anticorps humains de classe spécifique, qui ne peuvent pas se lier à l'antigène A, et comme solution 2 un récepteur R² qui est marqué et qui présente l'antigène A.

9. Réactif caractérisé en ce qu'il contient comme solution 1 un mélange d'un récepteur R¹ et d'un excès, par rapport à la quantité d'immunoglobuline à laquelle on doit s'attendre dans la solution échantillon, d'anticorps humains de classe spécifique, comme solution 2 un récepteur R² qui est marqué et qui contient un anticorps dirigé contre l'antigène A et comme solution 3 un récepteur R³ qui présente l'antigène A avec un moins un site de liaison pour l'anticorps à déterminer et un site de liaison pour le récepteur R².
